# EUROPÄISCHE PATENTSCHRIFT

(11) **EP 2 531 151 B1**
(45) Veröffentlichungstag und Bekanntmachung des Hinweises auf die Patenterteilung: **16.12.2015**
(21) Anmeldenummer: 11700896.1
(22) Anmeldetag: 21.01.2011
(51) Int. Cl.: A61F 5/058

(54) **SCHIENE ZUR RUHIGSTELLUNG EINES GELENKS**
SPLINT FOR IMMOBILISING A JOINT
ATTELLE D'IMMOBILISATION D'ARTICULATION

(30) Priorität: 04.02.2010 DE 102010001583
(43) Veröffentlichungstag der Anmeldung: 12.12.2012
(73) Patentinhaber: BSN medical GmbH, 20253 Hamburg (DE)
(72) Erfinder: KANDT, Olaf, 25474 Bönningstedt (DE)
(74) Vertreter: UEXKÜLL & STOLBERG
(86) Internationale Anmeldenummer: PCT/EP2011/000241
(87) Internationale Veröffentlichungsnummer: WO 2011/095282

(56) Entgegenhaltungen:
- EP-A1- 0 359 635
- EP-A1- 2 065 019
- US-A- 5 772 620

## Beschreibung

Die Erfindung betrifft eine Schiene zur Ruhigstellung eines Gelenks, die am ruhig zu stellenden Gelenk zu tragen ist, wobei die Schiene wenigstens einen Flächenkörper umfasst, der wenigstens zwei Auflageflächen für an das ruhig zu stellende Gelenk anschließende Gliedmaßen aufweist, wobei ein Stützkörper vorgesehen ist, der in seiner Außenkontur eine Winkelstruktur zur Einstellung und Stützung eines Winkels zwischen den Auflageflächen für die Gliedmaßen aufweist.

Bei Beanspruchung des Muskelapparats des menschlichen Körpers kommt es im Alltag, am Arbeitsplatz und in der Freizeit immer wieder zu Verletzungen, die es notwendig machen, dass diese unter Einschluss eines oder mehrerer Gelenke ruhig gestellt werden, um eine komplikationslose Ausheilung zu gewährleisten.

Verletzungen der Knochen, Sehnen, Muskeln, Nerven oder Blutgefäße gehen oftmals mit großen Schmerzen einher. Dies gilt insbesondere für Knochenbrüche und Weichteilverletzungen. Symptome hierbei sind Bewegungs-, Anspannungs-, Druck- und Dehnschmerzen sowie Blutungen. Besonders häufig sind Verletzungen im Breiten- und Leistungssport sowie im Alltag. Eine Vielzahl von in diesen Bereichen auftretenden Verletzungen und Schmerzzuständen erfordert eine Schienenversorgung.

Bei leichten Verletzungen und Überlastsyndromen sind neben dem Ruhigstellen auch Kälteanwendungen sowie das Hochlagern des betroffenen Körperteils üblich. Weiterhin kommen ruhig stellende Tapeverbände, Salbenverbände und Zinkleimverbände sowie physikalische Therapien, Orthesen sowie Bandagenversorgungen zum Einsatz.

Bei schweren Verletzungen oder Schmerzzuständen werden ausschließlich Schienenverbände angelegt. Dabei wird der betroffene Körperteil unter Einschluss eines oder mehrerer Gelenke durch Einsatz einer Schiene oder Fertigorthese ruhig gestellt. Üblicherweise werden hierzu Schienen aus Materialien wie Naturgips, thermoplastischem Kunststoff, Glasfasern oder Polyester sowie Fertigorthesen aus Kunststoff oder Aluminium verwendet.

Fertigorthesen und individuell anzufertigende Schienen unterscheiden sich darin, dass die Ruhigstellung eines oder mehrerer Gelenke nach dem Anlegen einer individuell anzufertigenden Schiene eine Fixierung mit einer Binde erfordert. Fertigorthesen werden dagegen mit Klettverschlusssystemen ausgestattet, die zur Fixierung dienen. Aufgrund der aufwendigeren Herstellung werden Fertigorthesen aus Kostengründen ausschließlich bei Langzeitversorgungen angewendet. Bei kurzfristigen Anwendungen kommen Schienen zum Einsatz, die vom Therapeuten selbst hergestellt und individuell angepasst werden.

Die Schienenversorgung ist zeitaufwendig und kostenintensiv, da eine individuelle Anfertigung und Anpassung notwendig ist. Wegen des hohen Materialgewichts in verarbeitungsbedingter scharfkantiger Formung sind solche Schienen oftmals mit einem schlechten Tragekomfort zu tragen. Das Anlegen und Herstellen solcher Schienenverbände, wie z.B. das Formen des Gipses in die medizinisch gewünschte Schiene ohne scharfe Kanten, erfordern besondere Fähigkeiten und Kenntnisse, welche gezielt ausgebildet und trainiert werden müssen.

Mit solchen bekannten Schienensystemen ist es nicht möglich, schnell am Ort einer gerade erlittenen Verletzung eine Schiene anzulegen, mit der das betroffene Gelenk bzw. Körperteil ruhig gestellt werden kann, um eine weitere Verschlimmerung der Verletzung kurzfristig zu verhindern. Auch sind solche Schienen nicht einsetzbar, wenn noch eine Versorgung von die Haut betreffenden Verletzungen notwendig ist, da sich herkömmliche Schienensysteme nicht flexibel abnehmen lassen, um danach wieder angebracht zu werden.

Aus der EP 2 065 019 A1 sind unterschiedliche Formen von aus flächigem Material hergestellten Schienen für den Arm bzw. das Handgelenk bekannt. Dabei wird ein Stützkörper zum Einstellen des Winkels zwischen Auflageflächen für den Unterarm und einen Teil der Hand beschrieben.

Die EP 0 359 635 A1 beschreibt eine Schiene für den Arm eines Patienten, die aus flächigem Material gebildet ist, wobei der Winkel zwischen Auflageflächen allein durch eine geeignete Verbindung von Abschnitten des flächigen Materials eingestellt wird.

Aus der U.S. 5,772,620 ist ebenfalls ein Stützkörper für den Unterarm eines Patienten bekannt, wobei der Stützkörper mit Hilfe von Gurten am Unterarm festgelegt wird und durch seine Form die Winkelstellung der einzelnen Abschnitte des Unterarms relativ zueinander festlegt.

Der Erfindung liegt die Aufgabe zugrunde, Schienen zur Ruhigstellung eines Gelenks, die am ruhig zu stellenden Gelenk zu tragen sind, zur Verfugung zu stellen, mit denen eine Sofortversorgung an einer Verletzung zur Ruhigstellung eines Gelenks erfolgen kann, die gleichzeitig einen erhöhten Tragekomfort bieten, kostengünstig herstellbar und konfektionierbar und mit einer erhöhten Stabilität ausgestattet sind.

Diese Aufgabe wird gelöst durch eine Schiene zur Ruhigstellung eines Knies gemäß Anspruch 1, die am ruhig zu stellenden Knie zu tragen ist, wobei die Schiene einen Flächenkörper umfasst, wobei der Flächenkörper zwei Auflageflächen für an das ruhig zu stellende Knie anschließende Gliedmaßen aufweist, wobei ein Stützkörper vorgesehen ist, der in seiner Außenkontur eine Winkelstruktur zur Einstellung und Stützung eines Winkels zwischen den Auflageflächen für die Gliedmaßen aufweist, dadurch gekennzeichnet, dass der Stützkörper an den von den Gliedmaßen abgewandten Seiten der Auflageflächen aus parallel zueinander angeordneten Flächenkörpern aufgebaut ist, die wenigstens doppelwandig oder mehrwandig verschiebefest zusammengefügt sind, und dass die Gelenkachse des ruhig zu stellenden Knies im Wesentlichen parallel zu einer Normalen auf die verschiebefest zusammengefügten Flächenkörper ist.

Die verschiebefeste Zusammenfügung mehrerer Flächen, die von der Unterseite der Auflageflächen abstehen, hat erfindungsgemäß die Wirkung, dass die Flächen nicht nur die Auflagefläche stärker stabilisieren, sondern sich auch gegenseitig stabilisieren. Dies führt dazu, dass der Stützkörper beispielsweise gegen seitliches Wegklappen geschützt ist, was der Stabilität der Schiene zugutekommt. Unter verschiebefest zusammengefügt wird verstanden, dass eine Verschiebung der Flächen gegeneinander erfindungsgemäß sowohl in Längsrichtung der Schiene als auch senkrecht zu den Auflageflächen unterbunden wird. Eine Fläche ist im Rahmen der Erfindung insbesondere ein Flächenkörper, der eine, insbesondere vorgebbare Dicke aufweist.

Im Rahmen der vorliegenden Erfindung bedeutet der Begriff "doppelwandig" oder "mehrwandig", dass zwei oder mehrere Flächen parallel zueinander mit keinem oder nur geringem Abstand angeordnet sind. Dies wird verdeutlicht an einem Fall, in dem der Stützkörper aus einem Flächenkörper zusammengefaltet wird und sich nach der Faltung ein im Wesentlichen rechteckiger Querschnitt ergibt. Der rechteckige Querschnitt eines solchen Stützkörpers hat zwei Außenwände, von denen jedoch wenigstens eine erfindungsgemäß wenigstens abschnittsweise doppelwandig oder mehrwandig ausgeführt ist.

Diese doppelwandige oder mehrwandige Ausführung der Seitenwände, d.h. der Wände, die von den Aufnahmeflächen nach unten abstehen, führt dazu, dass ein solcher im Wesentlichen rechteckiger Querschnitt nicht durch eine seitliche Kraft in einen rautenförmigen Querschnitt umgewandelt werden kann, der eine geringere Stützwirkung hätte.

Eine Normale einer Fläche ist eine Linie, die senkrecht auf der Fläche steht. Das Merkmal, dass eine Gelenkachse des ruhig zu stellenden Gelenks mit einer Normalen der verschiebefest zusammengefügten Flächen einen Winkel von weniger als 60° einschließt, bedeutet somit im Rahmen der Erfindung, dass die Gelenkachse die Flächen senkrecht oder in einem Winkel von bis zu 60° durchstößt, jedoch nicht in den Flächen liegt. Damit wird die Tatsache zur Stabilisierung des ruhig zu stellenden Gelenks ausgenutzt, dass die Flächen, insbesondere im zusammengefügten Zustand, eine hohe Steifigkeit gegenüber Scherkräften, also gegen Verbiegungen in der Fläche aufweisen, auch wenn sie gegenüber Verbiegungen, die senkrecht zur Fläche gerichtet sind, wenig steif sind. Vorzugsweise schließen die Gelenkachse des ruhig zu stellenden Gelenks oder eine Kante zwischen den Auflageflächen und die Normale der verschiebefest zusammengefügten Flächen einen Winkel von weniger als 45° ein, insbesondere vorzugsweise einen Winkel von weniger als 30°, weiter vorzugsweise einen Winkel von weniger als 15°. Vorzugsweise sind die Normale der verschiebefest zusammengefügten Flächen und die Gelenkachse bzw. die Kante oder die Schnittlinie im Wesentlichen parallel zueinander.

Dabei ist vorzugsweise vorgesehen, dass zwei Paare von wenigstens zwei im zusammengesetzten Zustand der Schiene an der von den Gliedmaßen abgewandten Seite der Auflageflächen angeordneten Flächen vorgesehen sind, die wenigstens abschnittsweise doppelwandig oder mehrwandig verschiebefest zusammengefügt sind, die symmetrisch um eine Längsachse der Schiene zueinander angeordnet sind. Dies ergibt eine symmetrische Dreiecks- oder Trapezform, die eine besonders hohe Stabilität der Schiene erzeugt.

Erfindungsgemäße Stützkörper können eine konkave oder eine konvexe Seite einer Schiene überbrücken oder seitlich dazu angeordnet sein.

In einer bevorzugten Ausführungsform weist der Stützkörper ein flächiges Material auf und ist in eine dreidimensionale Form gefaltet oder faltbar, die eine Außenkontur mit einer Winkelstruktur zur Formgebung für die Auflageflächen des Flächenkörpers aufweist. Eine solche gefaltete oder faltbare Struktur weist im Querschnitt eine Form auf, die eine Stützwirkung entfaltet, mit von der Unterseite der Auflageflächen abstehenden Flächen, d.h. Seitenflächen, die erfindungsgemäß wenigstens abschnittsweise doppelwandig oder mehrwandig ausgeführt sind. Die Grundformen, die sich hierfür besonders eignen, sind eine gleichschenklige Dreiecksform, eine symmetrische Trapezform oder eine Rechteckform.

In dieser erfindungsgemäßen Ausführungsform wird aus mehreren Flächenkörpern ein quasi-massiver Stützkörper erstellt, was bei geeigneten vorgefertigten Formen einfach und schnell möglich ist. Vorzugsweise ist der Stützkörper an einer Außenkontur mit einer Winkelstruktur mit einer von den Gliedmaßen abgewandten Seite oder Unterseite des Flächenkörpers verbindbar oder verbunden. Dabei ist im Rahmen der Erfindung die Unterseite eines Flächenkörpers definiert als die Seite des Flächenkörpers, die von den Gliedmaßen und vom ruhig zu stellenden Gelenk abgewandt ist. Es werden vorzugsweise auch mehrere Stützkörper, insbesondere parallel zueinander, verwendet. Diese können seitlich des ruhig zu stellenden Gelenks angeordnet sein.

Die Verbindung der Flächenkörper untereinander und/oder die Verbindung des Stützkörpers mit dem Flächenkörper umfasst vorzugsweise eine Verklebung, eine Vernietung, eine Verschraubung, eine Verklammerung, eine Verlaschung oder einen Klett-Verschluss. Unter einer Verlaschung wird in diesem Zusammenhang beispielsweise der Fall verstanden, dass ein abknickbares Flächenelement oder Steckelement einer flächigen Lasche eines Flächenkörpers durch einen entsprechenden Schlitz eines anderen Flächenkörpers geschoben wird und auf diese Weise eine Fixierung erreicht wird, die gegebenenfalls lösbar sein kann. Unter einer Verklebung wird im Rahmen der Erfindung auch eine Verleimung verstanden.

Der aus miteinander verbundenen Flächenkörpern aufgebaute Stützkörper ist vorzugsweise ausgestanzt, wobei insbesondere zunächst die Flächenkörper gestapelt und miteinander verbunden werden und anschließend der gesamte Stützkörper gestanzt wird. Auch vorgestanzte Flächenkörper können erfindungsgemäß zu einem Stützkörper zusammengefügt werden. Derart zusammengefügte, insbesondere verleimte, Stützkörper ergeben leichtgewichtige und höchst stabile Schienen.

Es werden vorzugsweise Stützkörper mit jeweils verschiedenen Winkeln für das ruhig zu stellende Gelenk hergestellt und/oder vorgehalten, so dass im Falle ihres Einsatzes die passende Winkelstellung für ein Gelenk anpassbar ist.

Ein aus zwei oder mehreren Lagen aufgebauter Stützkörper kann als Block oder geteilt, beispielsweise mit zwei kleineren Winkeln, an der Schiene angebracht werden. Dies ermöglicht eine bessere Stapelbarkeit bei der Lagerung.

In einer Weiterbildung der Erfindung ist vorzugsweise vorgesehen, dass der Stützkörper im gefalteten Zustand einen längs der Schiene erstreckten Hohlraum einschließt, in dem ein zweiter Stützkörper angeordnet oder in den ein zweiter Stützkörper einbringbar ist. Der erste Stützkörper, der gefaltet wird, und/oder der zweite Stützkörper, der in den Hohlraum einbringbar ist oder darin angeordnet ist, sind erfindungsgemäß doppelwandig oder mehrwandig ausgeführt, wie dieses vorstehend beschrieben ist. Eine solche doppelte Stützstruktur mit einem faltbaren ersten Stützkörper und einem quasimassiven oder massiven zweiten Stützkörper sorgt für eine nochmals erhöhte Stabilität der Stützstruktur.

Vorzugsweise bestehen der oder die Flächenkörper aus einem zugfesten Material, insbesondere aus Pappe oder Wellpappe. Diese Materialien sind in ihrer flächigen Ebene zugfest, jedoch um Falzlinien gut biegbar. Andere Materialien wie Kunststoffe, die in einer Richtung senkrecht zu ihrer Fläche elastischer sind, können auch ohne Falzlinien gebogen werden. Wenn der Flächen- oder Stützkörper als Wellpappe ausgeführt ist, sind die innenliegenden Rippen der Wellpappe vorzugsweise wenigstens teilweise orthogonal zur Biegung des Flächenkörpers bzw. des Stützkörpers oder wenigstens teilweise parallel zu den Falzlinien des Flächenkörpers und/oder des Stützkörpers ausgerichtet. Damit sind die Rippen wenigstens teilweise entlang des Rückens einer Biegung des Flächenkörpers ausgerichtet und unterstützen die stabilisierende Wirkung der Biegung. Wellpappe ist in der Richtung der Rippen gegen Verbiegung besonders stabil.

In einem aus mehreren miteinander verbundenen Lagen von Wellpappe aufgebauten Stützkörper sind die Wellen der verschiedenen Lagen der Wellpappe vorzugsweise parallel ausgerichtet. Um die Zugfestigkeit in verschiedene Richtungen zu erhöhen, ist weiterhin vorteilhafterweise vorgesehen, dass die Wellen der verschiedenen Lagen der Wellpappe in verschiedenen Richtungen ausgerichtet sind, insbesondere senkrecht zueinander, insbesondere in alternierender Abfolge von Lagen.

Vorzugsweise bestehen der Flächenkörper und der Stützkörper aus einem einstückigen Flächenkörper. Auf diese Weise sind gegebenenfalls sämtliche zu einer Schiene zusammensetzbare Teile mit einem Griff verfügbar.

Die erfindungsgemäßen Schienen sind beispielsweise durch Umwickeln mit Verbandmaterial oder durch Festzurren mit Riemen oder ähnlichen Fixiermitteln an den ruhig zu stellenden Gelenken bzw. den angrenzenden Körperteilen zu befestigen.

Stützkörper können erfindungsgemäß in der Beuge eines Gelenks, also beispielsweise einer Kniekehle, angeordnet sein, an seiner Außenseite, also beispielsweise entlang einer Kniescheibe, oder seitlich außerhalb. Jede dieser Versionen bietet Vorteile. Ein an der Kniescheibe angeordneter Stützkörper entfaltet seine Stützwirkung nahe am Knochen, während ein an der Kniekehle angeordneter Stützkörper an einer glatteren Fläche angreift.

Es kommt auch vor, dass wegen weiterer oberflächlicher Verletzungen ein Bereich des Gelenks oder der angrenzenden Gliedmaßen nicht für Stützmaßnahmen zur Verfügung steht. So ist es möglich, die Anordnung und Auswahl der Schiene und der Stützkörper an spezifische Verletzungsbilder und -situationen anzupassen.

Die Aufgabe wird ferner durch eine Schiene zur Ruhigstellung eines Gelenks, die am ruhig zu stellenden Gelenk zu tragen ist, gelöst, wobei die Schiene wenigstens einen Flächenkörper umfasst, wobei die Schiene wenigstens zwei Auflageflächen für die an das ruhig zu stellende Gelenk anschließenden Gliedmaßen aufweist, wobei ein Stützkörper vorgesehen ist, wobei der Stützkörper als Formkörper ausgebildet ist, dessen Winkel zwischen wenigstens zwei Formkörperflächen, die mit den abgewandten Seiten der Auflageflächen des Flächenkörpers verbunden sind, den Winkel zwischen den Auflageflachen vorgibt.

Vorzugsweise ist der Formkörper einstückig ausgebildet. Dieser kann allerdings auch zwei- oder mehrstückig sein. Im Falle dessen, dass der Formkörper zweistückig oder mehrstückig ist, sind die einzelnen Stücke vorzugsweise verbindbar, so dass diese einen Formkörper insgesamt bilden. Vorzugsweise weist der Formkörper wenigstens drei Formkörperflächen auf, die zum Flächenkörper hin orientiert sind, und wenigstens zwei Winkel des Flächenkörpers vorgeben. In diesem Fall können auch komplexere Strukturen im Hinblick auf deren Form vorgegeben werden. Vorzugsweise weist der Stützkörper in einer Außenkontur eine Winkelstruktur zur Einstellung und Stützung eines Winkels zwischen den Auflageflächen für die Gliedmaßen auf.

Vorzugsweise ist der Formkörper massiv ausgebildet, was eine schnelle und kostengünstige Fertigung ermöglicht. Vorzugsweise ist der Formkörper aus einem einzigen Material ausgebildet. Vorzugsweise liegt die Dichte des Formkörpers in einem Bereich von 0,01 g/cm³ bis 3 g/cm³, insbesondere bis 0,5 g/cm³. Der Formkörper ist entsprechend auch vorzugsweise mit dem Flächenkörper oder den Flächenkörpern über eine Verklebung, Vernietung, Verschraubung, Verklammerung, Verlaschung oder einen oder mehrere Klettverschlüsse verbunden. Unter massiv wird insbesondere im Sinne der Erfindung auch verstanden, dass mehrere Lagen Pappe bzw. Wellpappe miteinander verbunden vorliegen.

Der Formkörper kann beispielsweise aus Balsaholz sein oder einem Kunststoffschaum, vorzugsweise einem geschlossenporigen Kunststoffschaum, sein. Hierzu eignen sich insbesondere Hartschaume wie Polyurethan-Hartschaum, Polyethylen-Hartschaum, Polycarbodiimid-Hartschaum oder ähnliche Materialien. Hierbei ist es bevorzugt, wenn die Druckfestigkeit zwischen 0,3 und 15 MPa ist. Die Druckfestigkeit und/oder die Zugfestigkeit des Formkörpers bzw. des Stützkörpers sollten so ausgebildet sein, dass eine ausreichende Stabilität der Schiene für das betreffende Kniegelenk ermöglicht ist. Für den Fachmann ist es möglich, anhand der auftretenden Belastungen an dem jeweiligen Gelenk die Druckfestigkeit oder die Zugfestigkeit zu bestimmen.

Der Formkörper kann zudem aus einem Kompositmaterial bestehen, beispielsweise aus einem Kompositmaterial aus einem Kunststoffschaum mit einem Metall, das wenigstens teilweise den Kunststoffschaum ummanteln kann. Als Metall eignet sich hierbei beispielsweise Aluminium oder Titan. Das Kompositmaterial liegt vorzugsweise in einer Sandwichstruktur vor. Der Formkörper hat vorzugsweise auch zwei Außenflächen, deren Normale einen Winkel von 60° mit einer Gelenkachse oder einer Kante zwischen den Auflageflächen des Flächenkörpers einschließt.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische Querschnittsdarstellung durch einen erfindungsgemäßen Flächenkörper,
- Fig. 2: schematische Querschnittsdarstellungen durch weitere erfindungsgemäße Flächenkörper,
- Fig. 3: eine schematische Draufsicht auf einen Flächenkörper für eine erfindungsgemäße Schiene für ein Kniegelenk,
- Fig. 4: eine schematische Seitendarstellung einer erfindungsgemäßen Schiene gemäß Fig. 10,
- Fig. 5: eine schematische Seitendarstellung einer weiteren erfindungsgemäßen Schiene für ein Kniegelenk und
- Fig. 6: eine schematische Seitendarstellung einer weiteren erfindungsgemäßen Schiene für ein Kniegelenk.

In den folgenden Figuren sind jeweils gleiche oder gleichartige Elemente bzw. entsprechende Teile mit denselben Bezugsziffern versehen, so dass von einer entsprechenden erneuten Vorstellung abgesehen wird.

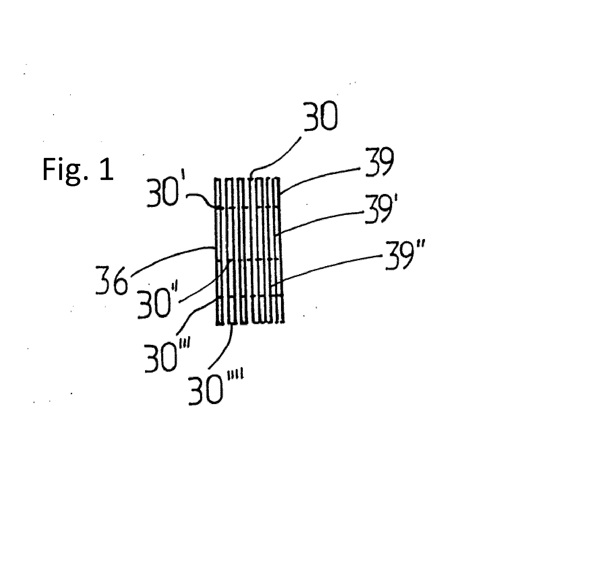

In Fig. 1 ist im Querschnitt schematisch gezeigt, dass ein entsprechender Stützkörper 36 aus einer Mehrzahl von aufeinander gestapelten Flächenkörpern 39, 39', 39" usw. aufgebaut ist. Die quer laufenden gestrichelten Linien entsprechen den Eckpunkten bzw. Kanten 30 bis 30"" der Struktur. Jeder einzelne vertikale Strich stellt in Fig. 1 eine schematische Repräsentation eines Flächenkörpers dar. Die Flächenkörper 39, 39', 39" usw. sind in dem Ausführungsbeispiel gemäß Fig. 1 miteinander verklebt. In diesem Fall schließt eine Normale auf die Flächenkörper 39, 39', 39", die in der horizontalen Bildebene verläuft, einen Winkel von 0° mit der Gelenkachse des Handgelenks ein.

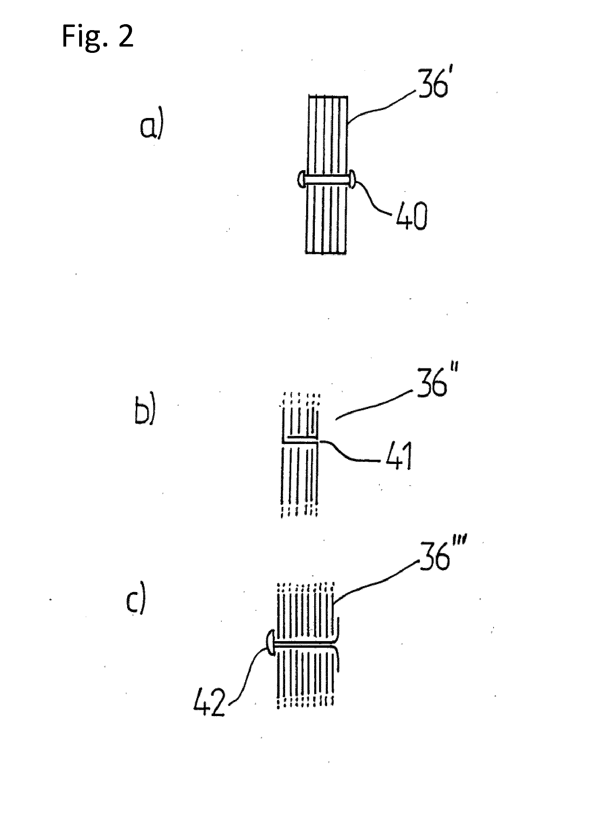

In Fig. 2a) bis 2c) sind drei verschiedene Beispiele dargestellt, wie entsprechende Flächenkörper in einem Stützkörper miteinander verbunden werden können. Fig. 2a) zeigt einen Stützkörper 36' mit einigen Flächenkörpern, die durch eine Niete 40 verbunden sind. Es können auch mehrere Nieten 40, die jeweils zwei Köpfe aufweisen, verwendet werden.

In Fig. 2b) weist ein Stützkörper 36" eine Verlaschung 41 auf. Dies bedeutet, dass zwei außen gelegene Flächenkörper jeweils eine Lasche aufweisen, die durch einen Schlitz in den innen liegenden Flächenkörpern geführt wird und an der anderen Seite wiederum gebogen bzw. gefaltet und weitergeführt wird. Dies kann kreuzweise von beiden Seiten aus geschehen oder in geeigneter anderer Anordnung.

Fig. 2c) zeigt anhand eines weiteren Stützkörpers 36'" mit einer Mehrzahl von Flächenkörpern eine besonders einfache Verbindung mittels einer Rundkopfklammer 42 mit zwei Füßen, wobei jeder der Flächenkörper in dem Stützkörper 36'" an der gleichen Stelle ein Loch für eine Rundkopfklammer aufweist, die durch das entstehende Loch gesteckt wird. Am gegenüberliegenden Ende werden die Füße der Rundkopfklammer auseinandergebogen, so dass ein einfacher und sicherer Halt herstellbar ist.

Die in den Fig. 1 und 2 gezeigten Schienen und Stützkörper stellen Beispiele für einfach aus Flächenkörpern herstellbare und sehr stabile Schienen und Stützkörper dar. Diese sind leicht und schnell herstellbar und konfektionierbar.

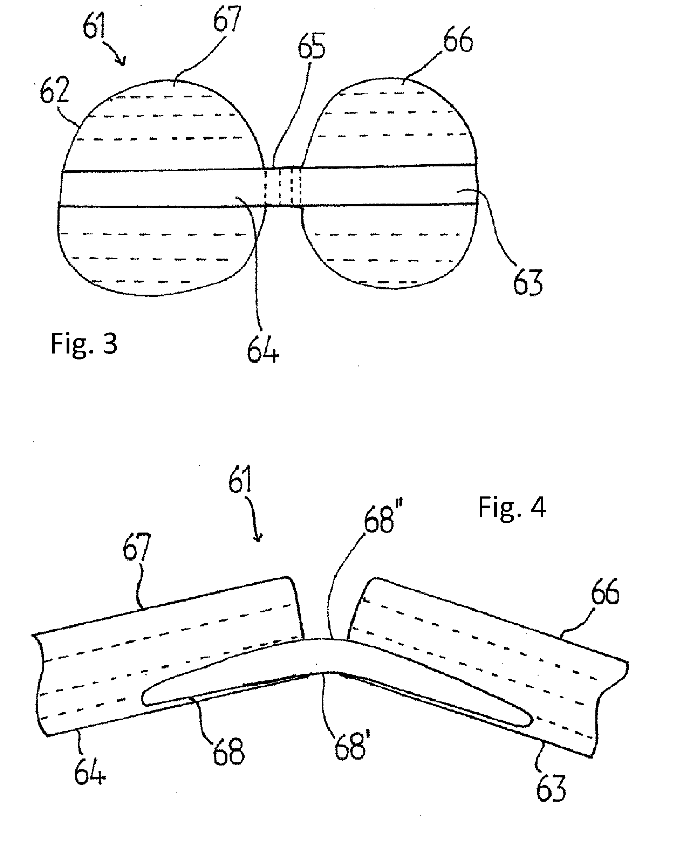

Fig. 3 stellt eine schematische Draufsicht auf einen Flächenkörper 62 für eine erfindungsgemäße Schiene 61 für ein Kniegelenk dar. Ein bevorzugter Beugungswinkel für ein ruhig zu stellendes Kniegelenk liegt bei ca. 20°.

Der Flächenkörper 62 weist eine Auflagefläche 63 für einen Oberschenkel und eine Auflagefläche 64 für einen Unterschenkel auf, an die sich seitlich Flächen 66, 67 für den Ober- bzw. Unterschenkel anschließen. In der Mitte ist eine quer gefalzte oder perforierte Auflagefläche 65 für eine Kniekehle angeordnet, die um die Querfalzlinien bzw. -perforationen biegbar ist, um eine Kniebeugung um einen einzustellenden Winkel nachzuvollziehen.

In Fig. 4 ist die eine Schiene 61 aus Fig. 3 schematisch von der Seite dargestellt. Ein Stützkörper 68, der wie in Fig. 1 und 2 dargestellt ausgebildet sein kann, ist seitlich mit dem Flächenkörper 62 verbunden, insbesondere mit den Seitenflächen 66, 67. Die Normale auf den Stützkörper 68 ist im Wesentlichen parallel zur Kniegelenkachse oder weist einen Winkel von bis zu 45° oder 60° zur Kniegelenkachse auf. Die gewinkelten Außenkonturen des Stützkörpers 68 sind mit den Bezugszeichen 68', 68" versehen.

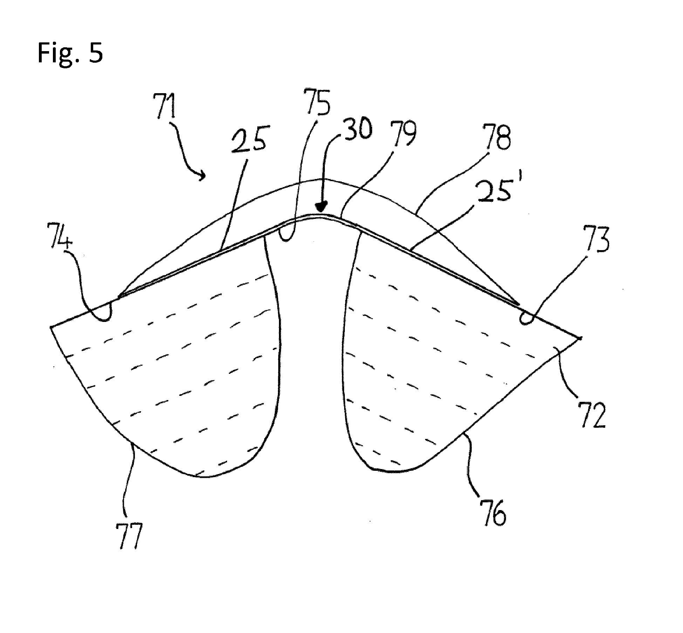

Fig. 5 zeigt schematisch von der Seiten eine alternative erfindungsgemäße Schiene 71 für ein Kniegelenk. Ein Flächenkörper 72 mit Auflageflächen 73, 74, 75 für einen Oberschenkel, einen Unterschenkel und eine Kniescheibe ist ausgebildet, um an eine Außenseite eines Kniegelenks angelegt zu werden. Seitenflächen 77, 78 werden um die angrenzenden Gliedmaßen gefaltet. An die Außenseite des Flächenkörpers 72, also an der vom Kniegelenk abgewandten Seite, ist ein Stützkörper 78 angesetzt, der eine konkave Innenfläche als Außenkontur 79 aufweist. Mit der konkaven Innenfläche ist der Stützkörper 78 mit der Außenseite des Flächenkörpers 72 verbunden. Die Außenkontur 79 weist eine abgerundete Kante 30 auf. Die gedachten Verlängerungen der Außenkontur 79 bilden einen Winkel, der den Winkel zwischen den Auflageflächen 73 und 74 vorgibt.

Der Stützkörper 78 ist aus parallelen miteinander verbundenen Flächenkörpern, wie in Fig. 1, 2 gezeigt, aufgebaut, wobei die Normale auf die Flächenkörper parallel zur Kniegelenkachse ausgerichtet ist. Alternativ können auch die Stützkörper 68 aus Fig. 4 so verwendet werden, dass sie in etwa in der Breite einer Kniekehle aneinander geklebt werden und in die Kniekehle eingelegt werden. Insbesondere können die Stützkörper 68 aus Fig. 4 und die Stützkörper 78 aus Fig. 5 die gleiche Form haben und alternativ in den in Fig. 4 und Fig. 5 gezeigten Stellungen verwendet werden. Dabei ist vorzugsweise die konvexe Seite an die Form einer Kniekehle angepasst und die konkave Seite an die Form der Außenseite eines Knies mit einer Kniescheibe.

In den in den Figuren gezeigten Ausführungsbeispielen kann anstelle eines mittig angeordneten Stützkörpers auch ein Paar oder eine Mehrzahl von im Wesentlichen parallel angeordneten und/oder ausgerichteten Stützkörpern verwendet werden.

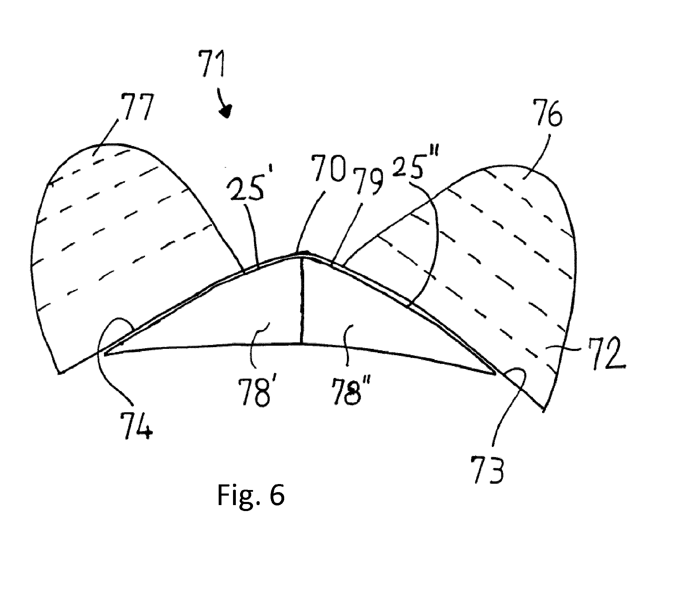

Fig. 6 zeigt eine schematische Seitendarstellung einer weiteren erfindungsgemäßen Schiene 71 für ein Kniegelenk. Hier ist die Auflagefläche 73 für einen Oberschenkel und die Auflageflache 74 für einen Unterschenkel so ausgebildet, dass eine Kniekehle auf diesen Flächen aufliegen kann. Hierzu ist auch die Bezugsziffer 70 als Auflagefläche für eine Kniekehle vorgesehen. Mittels der seitlichen Flächen 76 und 77 für den Oberschenkel und den Unterschenkel können diese entsprechend fixiert werden. Um einen entsprechenden Winkel zwischen den Auflageflächen 73 und 74 vorzugeben, sind zwei Stützkörper 78' und 78" vorgesehen, die in mit dem Flächenkörper 72 verbundener Form aneinanderliegend angeordnet sind. Die beiden Stützkörper 78' bzw. 78" können auch als Formkörper bezeichnet werden und weisen eine dreidimensionale Form auf, die in etwa die Tiefe der Auflageflachen 73 und 74 haben können. Die angrenzenden Flächen der Stützkörper 78 und 78' zueinander können auch miteinander verklebt sein oder mit einer anderen Verbindung wie beispielsweise Klettverschluss verbunden sein. Die Außenkontur 79 der Stützkörper 78' und 78" weisen Formkörperflächen 25' und 25" auf, die mit dem Flächenkörper 72 verbunden sind. Die Stützkörper 78' und 78" sowie der Flächenkörper 72 können getrennt voneinander gelagert werden und benötigen so wenig Packmaß. In der Anwendung können diese dann miteinander verbunden werden.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen.

### Bezugszeichenliste

- 1: Schiene
- 2: Flächenkörper
- 3: Auflagefläche für einen Unterarm
- 4: Auflagefläche für einen Handballen
- 4': Auflagefläche für Daumen
- 5: Auflagefläche für Finger
- 6: Stützkörper
- 7: Außenkontur
- 8, 8': Winkelstruktur
- 9: Unterseite
- 10, 10': Seitenfläche
- 11, 11': Oberseite
- 12, 12': Unterseitenfalzlinie
- 13, 13': Schlitz
- 14, 15, 16: Querfalz
- 17, 17': Lasche
- 18, 18': Laschenfalzlinie in der Auflagefläche
- 19, 19': Laschenfalzlinie in der Lasche
- 20, 20': Längsfalzlinie
- 21: Aussparung für Lasche
- 22, 22': Laschensteckteil
- 25, 25', 25": Formkörperfläche
- 30, 30', 30",30"', 30"": Kante
- 31: Schiene
- 32: Flächenkörper
- 33: Auflagefläche für einen Unterarm
- 34: Auflagefläche für einen Handballen
- 34': Auflagefläche für Daumen
- 35: Auflagefläche für Finger
- 36 - 36"': Stützkörper
- 37: Außenkontur
- 38, 38': Winkelstruktur
- 39 - 39": Flächenkörper
- 40: Niete
- 41: Verlaschung
- 42: Rundkopfklammer
- 51: Schiene
- 52, 52': Flächenkörper
- 53: Auflagefläche für einen Oberarm
- 54: Auflagefläche für einen Unterarm
- 56, 56': Stützkörper
- 57, 57': Außenkontur
- 61: Schiene
- 62: Flächenkörper
- 63: Auflagefläche für einen Oberschenkel
- 64: Auflagefläche für einen Unterschenkel
- 65: Auflagefläche für eine Kniekehle
- 66: seitliche Fläche für Oberschenkel
- 67: seitliche Fläche für Unterschenkel
- 68: Stützkörper
- 68', 68": Außenkontur
- 70: Auflagefläche für Kniekehle
- 71: Schiene
- 72: Flächenkörper
- 73: Auflagefläche für einen Oberschenkel
- 74: Auflagefläche für einen Unterschenkel
- 75: Auflagefläche für eine Kniescheibe
- 76: seitliche Fläche für Oberschenkel
- 77: seitliche Fläche für Unterschenkel
- 78, 78', 78": Stützkörper
- 79: Außenkontur
- 80: Falzlinie

## Patentansprüche

1. Schiene (61, 71) zur Ruhigstellung eines Knies, die am ruhig zu stellenden Knie zu tragen ist, wobei die Schiene (61, 71) einen Flächenkörper (62, 72) umfasst, wobei der Flächenkörper (62, 72) zwei Auflageflächen (63, 64, 73, 74) für an das ruhig zu stellende Knie anschließende Gliedmaßen aufweist, wobei ein Stützkörper (68, 78) vorgesehen ist, der in seiner Außenkontur (68', 68", 79) eine Winkelstruktur zur Einstellung und Stützung eines Winkels zwischen den Auflageflächen (63, 64, 73, 74) für die Gliedmaßen aufweist, **dadurch gekennzeichnet, dass** der Stützkörper (68, 78) an den von den Gliedmaßen abgewandten Seiten der Auflageflächen (63, 64, 73, 74) aus parallel zueinander angeordneten Flächenkörpern (39, 39', 39") aufgebaut ist, die wenigstens doppelwandig oder mehrwandig verschiebefest zusammengefügt sind, und dass die Gelenkachse des ruhig zu stellenden Knies im Wesentlichen parallel zu einer Normalen auf die verschiebefest zusammengefügten Flächenkörper ist.

2. Schiene (61, 71) nach Anspruch 1, **dadurch gekennzeichnet, dass** der Stützkörper (6) ein flächiges Material aufweist und in eine dreidimensionale Form gefaltet ist, die eine Außenkontur mit einer Winkelstruktur (68, 68', 78, 78') zur Formgebung für die Auflageflächen (63, 64, 73, 74) des Flächenkörpers (62, 72) aufweist.

3. Schiene (61, 71) nach Anspruch 1 oder 2, **dadurch gekennzeichnet, dass** der Stützkörper an einer Außenkontur mit einer Winkelstruktur mit einer von den Gliedmaßen abgewandten Seite des Flächenkörpers (62, 72) verbunden ist.

4. Schiene (61, 71) nach einem der Anspruche 1 bis 3, **dadurch gekennzeichnet, dass** der oder die Flächenkörper (2, 32, 39, 39', 39") aus einem zugfesten Material, insbesondere aus Pappe oder Wellpappe bestehen.

5. Schiene (61, 71) nach einem der Ansprüche 1 bis 4, **dadurch gekennzeichnet, dass** der Flächenkörper (2, 32) und der Stützkörper (6) aus einem einstückigen Flächenkörper bestehen.

6. Schiene (61, 71) nach einem der Anspruche 1 bis 5, **dadurch gekennzeichnet, dass** die Verbindung der Flächenkörper (39, 39', 39") untereinander und/oder des Stützkörpers (68, 78) mit dem Flächenkörper (62, 72) eine Verklebung, Vernietung, Verschraubung, Verklammerung, Verlaschung oder einen Klett-Verschluss umfasst.

## Claims

1. A splint (61, 71) for immobilization of a knee, which is to be worn on the knee to be immobilized, wherein the splint (61, 71) comprises a flat body (62, 72), the flat body (62, 72) having two contact surfaces (63, 64, 73, 74) for limbs adjoining the knee to be immobilized, wherein a supporting member (68, 78) is provided, which in its outer contour (68', 68", 79) has an angle structure for adjusting and bracing an angle between the contact surfaces (63, 64, 73, 74) for the limbs, **characterized in that** the supporting member (68, 78) on the sides of the contact surfaces (63, 64, 73, 74) facing away from the limbs is formed of flat bodies (39, 39', 39") arranged in parallel to one another, which are immovably joined together to form at least a double-wall or a multiple-wall structure, and that the joint axis of the knee to be immobilized is substantially in parallel to a normal of the flat bodies immovably joined together.

2. The splint (61, 71) according to claim 1, **characterized in that** the supporting member (6) has a sheetlike material and is folded into a three-dimensional form having an outer contour with an angle structure (68, 68', 78, 78') for configuring the contact surfaces (63, 64, 73, 74) of the flat body (62, 72).

3. The splint (61, 71) according to claims 1 or 2, **characterized in that** the supporting member is connected at an outer contour with an angle structure to a side of the flat body (62, 72) facing away from the limbs.

4. The splint (61, 71) according to one of claims 1 to 3, **characterized in that** the supporting member or supporting members (2, 32, 39, 39', 39") consist of a high tensile strength material, especially cardboard or corrugated cardboard.

5. The splint (61, 71) according to one of claims 1 to 4, **characterized in that** the flat body (2, 32) and the supporting member (6) consist of a single-piece flat body.

6. The splint (61, 71) according to one of claims 1 to 5, **characterized in that** the connection of the flat bodies (39, 39', 39") to each other and/or of the supporting member (68, 78) to the flat body (62, 72) comprises an adhesive, riveting, screwing, clamping, strapping or a hook-and-eye connection.

## Revendications

1. Atelle (61, 71) pour l'immobilisation d'un genou, qui doit être porté au niveau d'un genou à immobiliser, l'atelle (61, 71) comprenant un corps plat (62, 72), le corps plat (62, 72) comprenant deux surfaces d'appui (63, 64, 73, 74) pour le membre raccordé au genou à immobiliser, un corps de soutien (68, 78) étant prévu, qui présente, sur son contour extérieur (68', 68", 79), une structure angulaire pour le réglage et le soutien d'un angle entre les surfaces d'appui (63, 64, 73, 74) pour les membres, **caractérisé en ce que** le corps de soutien (68, 78) est constitué, au niveau des côtés des surfaces d'appui (63, 64, 73, 74) opposés aux membres, de corps plats (39, 39', 39") disposés parallèlement entre eux, qui sont assemblés, de manière solidaire en coulissement, afin de former au moins une double paroi ou de multiples parois, et **en ce que** l'axe d'articulation du genou à immobiliser est essentiellement parallèle à une normale aux corps plats assemblés de manière solidaire en coulissement.

2. Atelle (61, 71) selon la revendication 1, **caractérisé en ce que** le corps de soutien (6) présente un matériau plat et est plié en une forme tridimensionnelle qui présente un contour extérieur avec une structure angulaire (68, 68', 78, 78') pour le façonnage pour les surfaces d'appui (63, 64, 73, 74) du corps plat (62, 72).

3. Atelle (61, 71) selon la revendication 1 ou 2, **caractérisé en ce que** le corps de soutien est relié, au niveau d'un contour extérieur, avec une structure angulaire avec un côté du corps plat (62, 72) opposé aux membres.

4. Atelle (61, 71) selon l'une des revendications 1 à 3, **caractérisé en ce que** le ou les corps plats (2, 32, 39, 39', 39") sont constitués d'un matériau résistant à la traction, plus particulièrement de carton ou de carton ondulé.

5. Atelle (61, 71) selon l'une des revendications 1 à 4, **caractérisé en ce que** le corps plat (2, 32) et le corps de soutien (6) sont constitués d'un corps plat d'une seule pièce.

6. Atelle (61, 71) selon l'une des revendications 1 à 5, **caractérisé en ce que** la liaison des corps plats (39, 39', 39") entre eux et/ou du corps de soutien (68, 78) avec le corps plat (62, 72) comprend un collage, un rivetage, une visserie, un agrafage, un éclissage ou une fermeture autoagrippante.
